# EUROPEAN PATENT SPECIFICATION

(11) **EP 3 011 340 B1**
(45) Date of publication and mention of the grant of the patent: **22.11.2017**
(21) Application number: 14729929.1
(22) Date of filing: 16.06.2014
(51) Int. Cl.: G01N 33/68, C07K 14/52, G01N 33/74

(54) **MRG RECEPTOR MODULATORS**
MRG-REZEPTORMODULATOREN
MODULATEURS DES RÉCEPTEURS MRG

(30) Priority: 18.06.2013 EP 13172485
(43) Date of publication of application: 27.04.2016
(73) Proprietor: Bayer Pharma Aktiengesellschaft, 13353 Berlin (DE)
(72) Inventor: GOLZ, Stefan, 45472 Mülheim (DE); MICUS, Sina, 42109 Wuppertal (DE)
(74) Representative: BIP Patents
(86) International application number: PCT/EP2014/062529
(87) International publication number: WO 2014/202515

(56) References cited:
- EP-A2- 1 340 979
- WO-A1-2013/073698

## Description

### Technical field of the invention

The present invention is in the field of molecular biology, more particularly, the present invention relates to drug screening, assay development and identification and use of modulators for G-protein coupled receptor/ligand pair MRGX2/CXCL14 within drug discovery.

### Background of the invention

### Cytokines

Chemoattractant cytokines or chemokines constitute a family of structurally related proteins found in vertebrates, bacteria, or viruses. So far, 48 chemokine genes have been identified in humans, which bind to around 20 chemokine receptors.These receptors belong to the seven transmembrane G-protein-coupled receptor family. Chemokines and their receptors were originally studied for their role in cellular trafficking of leukocytes during inflammation and immune surveillance. It is now known that they exert different functions under physiological conditions such as homeostasis, development, tissue repair, and angiogenesis but also under pathological disorders including tumorigenesis, cancer metastasis, inflammatory, and autoimmune diseases. Physicochemical properties of chemokines and chemokine receptors confer the ability to homo- and hetero-oligomerize. Many efforts are currently performed in establishing new therapeutically compounds able to target the chemokine/chemokine receptor system. In this review, we are interested in the role of chemokines in inflammatory disease and leukocyte trafficking with a focus on vascular inflammatory diseases, the operating synergism, and the emerging therapeutic approaches of chemokines [4].

### CXCL14

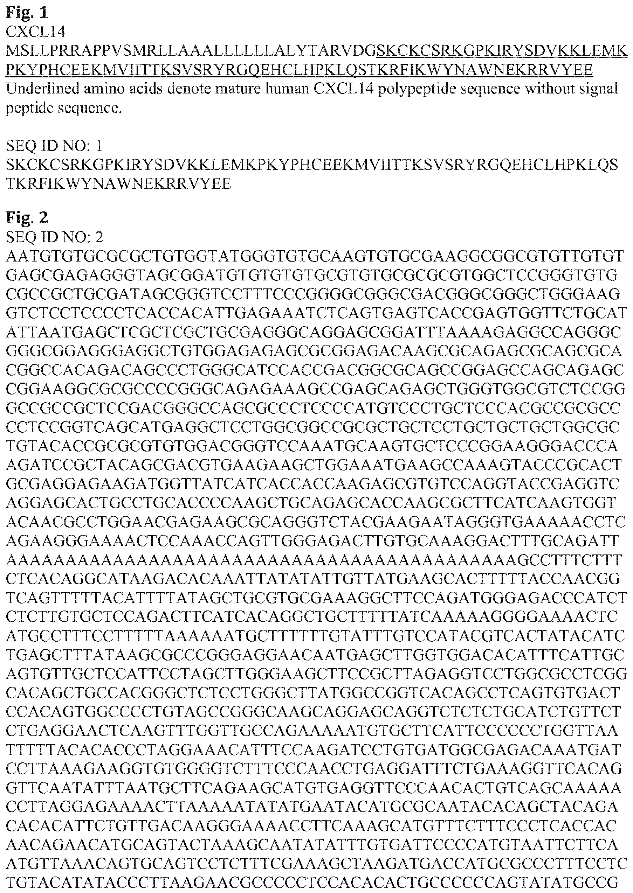

Chemokine (C-X-C motif) ligand 14 (CXCL14) is a small cytokine belonging to the CXC chemokine family that is also known as BRAK (for breast and kidney-expressed chemokine) [10]. Mature CXCL14 has many of the conserved features of the CXC chemokine subfamily but has some differences too, such as a shorter N-terminus and five extra amino acids in the region between its third and fourth cysteines. CXCL14 is constitutively expressed at high levels in many normal tissues, where its cellular source is thought to be fibroblasts. However, it is reduced or absent from most cancer cells. This chemokine is chemotactic for monocytes and can activate these cells in the presence of an inflammatory mediator called prostaglandin-E2 (PGE2). It is also a potent chemoattractant and activator of dendritic cells, is implicated in homing of these cells, and can stimulate the migration of activated NK cells. CXCL14 also inhibits angiogenesis, possibly as a result of its ability to block endothelial cell chemotaxis [11]. The gene for CXCL14 contains four exons and is located on chromosome 5 in humans. A receptor for CXCL14 was not described so far. The sequences of human CXCL14 (with and without signal peptide (amino acids 1 to 34)) are shown in fig. 1. The polypeptide of human CXCL14 is given in SEQ ID NO: 1 and the polynucleotide of human CXCL14 is presented in SEQ ID NO: 2 (see fig.s 1 and 2).

CXCL14-deficient mice do not exhibit clear immune system abnormalities. CXCL14 suppresses the in vivo growth of lung and head-and-neck carcinoma cells, whereas the invasiveness of breast and prostate cancer cells appears to be promoted by CXCL14. Moreover, recent evidence revealed that CXCL14 participates in glucose metabolism, feeding behaviour-associated neuronal circuits, and anti-microbial defense. Based on the expression patterns of CXCL14 and CXCL12 during embryonic development and in the perinatal brain in mice, the functions of these two chemokines may be opposite or interactive [22].

Previously, CXCL14 was demonstrated to be a highly selective chemoattractant for human monocytes pre-treated with prostaglandin E2 (PGE2) or forskolin [24]. However, later studies revealed that human monocyte-derived iDCs, but not mature dendritic cells, are responsive to CXCL14 in the absence of PGE2 treatment. In humans, CXCL14 is a chemoattractant for iDCs isolated from peripheral blood or induced in vitro from CD14Þ monocytes or CD34Þ hematopoietic progenitor cells [25, 26, 27]. CXCL14 up-regulates the expression of dendritic cell maturation markers and enhances the proliferation of allogenic T cells in mixed lymphocyte reactions. Therefore, chemoattraction of iDCs and functional maturation of dendritic cells by CXCL14 would substantially contribute to anti-tumour immune surveillance. In addition, natural killer (NK) cells have also been reported to be chemoattracted by CXCL14. Very recently, it was reported that CXCL14-transgenic mice exhibit a higher incidence of collagen-induced arthritis due to an enhanced Thl response and elevated levels of auto-antibodies [28]. The total numbers of lymphocytes, dendritic cells and macrophages were not significantly changed in these CXCL14-transgenic mice, again suggesting that CXCL14 is dispensable for steady-state immunity [22].

Furthermore CXCL14 is described as involved in cancer progression e. g. metastasis and angiogenesis. It is described that CXCL14 is involved in the SDF1a-CXCR4 pathway [40] and as an anti-cancer gene [41-43].

### Diseases associated with CXCL14

CXCL14 is described in literature to be possibly associated with diseases like (but not limited to): cancer [12, 15, 19], pulmonary fibrosis [13], infection and bactericidal activity [14, 22], kidney disease [16], macula disease [17], colitis [18], angiogenesis [26], arthritis [28], diabetes (glucose metabolism) [22], asthma [20], COPD [83], pulmonary embolism [84], cns disorders [85], cancer and diseases associated with the modulation of the SDF1a-CXCR4 pathway, mast cell activity, dorsal root ganglia function or inflammation.

Though, the chemokine CXCL14 is involved in various serious diseases the cognate receptor has not been described so far. However, for drug discovery and development of pharmaceuticals it is of outmost importance to know the identity of the receptor of a given ligand e.g. CXCL14 to identify and study modulators i.e. agonists or antagonists of such receptor-ligand interaction.

However, with the present invention we describe the identification of the G-protein coupled receptor MrgX2 as the cognate receptor for the chemokine CXCL14. This opens for the first time the door for drug discovery of MrgX2/CXCL14 receptor-ligand pair modulators.

### Summary of the invention

The invention is defined in the claims. The invention relates to the use of CXCL14 as a ligand for MrgX2 within drug discovery.

The invention relates to the use of CXCL14 for the characterization of MrgX2 modulators within drug discovery.

The invention relates to the use of CXCL14 for the characterization of Mrg-family member modulators within drug discovery.

The invention relates to the use of CXCL14 for the characterization of MrgX2 orthologue receptors within drug discovery.

The invention relates to the use of CXCL14 antibodies for the characterization of MrgX2 and its orthologues within drug discovery.

The invention relates to the use of CXCL14 in cell based MrgX2 assays.

The invention relates to the use CXCL14 in cell-free MrgX2 assays.

The invention relates to the use of CXCL14 in in vitro MrgX2 assays, as, but not limited to binding assays, activity assays, cell based assays and cell-free assays.

The invention relates to the use of CXCL14 assays to characterize compound, peptides or antibodies which modify the activity of MrgX2.

### Brief Description of the Drawings

Fig. 1: Fig. 1 shows the amino acid sequence of human CXCL14 precursor (including signal peptide from amino acid 1 to 34)
Fig. 2: Fig. 2 shows the nucleotide sequence of human CXCL14 precursor (including coding sequence for signal peptide)

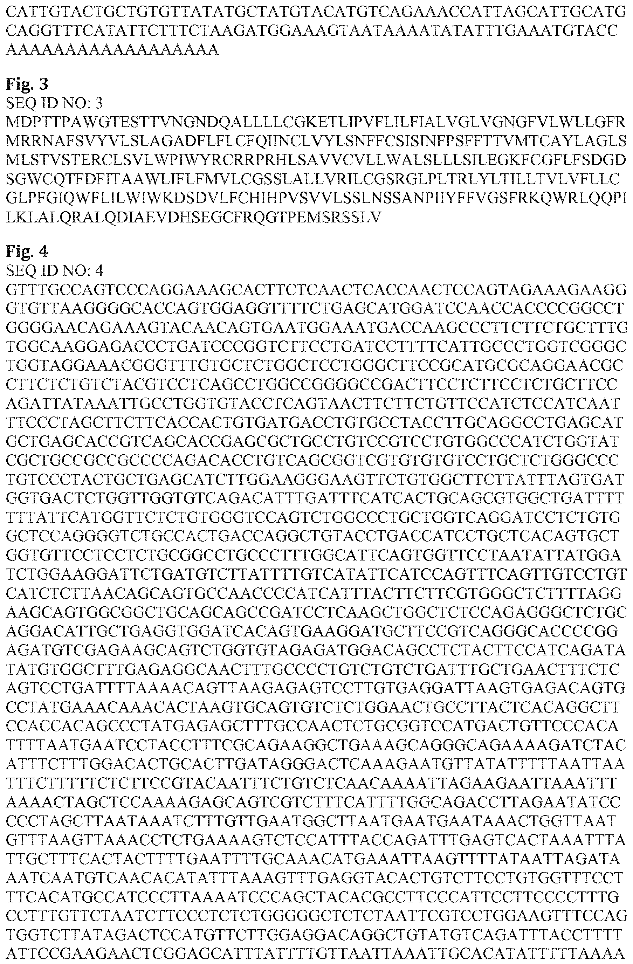

Fig. 3: Fig. 3 shows the amino acid sequence of human MrgX2
Fig. 4: Fig. 4 shows the nucleotide sequence of human MrgX2
Fig. 5: Fig. 5 gives an overview about some (but not limited to) functions of mast cell activation (Fig. from [79])
Fig. 6: Fig. 6 shows a table with identical amino acids (in %) of human and rat CXCL14
Fig. 7: Fig. 7 shows the dose response curve of CXCL14 activating MrgX2 in a cell based assay format (Fluo8 assay) (RFU: relative fluorescence units).
Fig. 8: Fig. 8 shows the dose response curve of CXCL14 on CHEM1 cells (Fluo8 assay) (RFU: relative fluorescence units).
Fig. 9: Fig. 9 shows the dose response curve of PAMP activating MrgX2 in a cell based assay format (Fluo8 assay) (RFU: relative fluorescence units).
Fig. 10: Fig. 10 shows the dose response curve of PAMP on CHEM1 cells (Fluo8 assay) (RFU: relative fluorescence units)
Fig. 11: Fig. 11 shows the activity of different peptides on MrgX2 in a cell based assay format (Fluo8 assay) (RFU: relative fluorescence units).
Fig. 12: Fig. 12 shows the results from expression analysis of Chem1 and Chem1-MrgX2 cells (y-axis: relative expression, x-axis: cell line)
Fig. 13: Fig. 13 shows nucleotide sequence of forward primer human MrgX2
Fig. 14: Fig. 14 shows nucleotide sequence of probe primer human MrgX2
Fig. 15: Fig. 15 shows nucleotide sequence of reverse primer human MrgX2
Fig. 16: Fig. 16 shows nucleotide sequence of forward primer human L32
Fig. 17: Fig. 17 shows nucleotide sequence of probe primer human L32
Fig. 18: Fig. 18 shows nucleotide sequence of reverse primer human L32
Fig. 19: Fig. 19 shows the amino acid sequence of mouse CXCL14
Fig. 20: Fig. 20 shows the network of CXCL14 with its receptors and selected pathways and disease associations
Fig. 21: Fig. 21 shows the amino acid sequence of CXCL14 fragment 1
Fig. 22: Fig. 22 shows the amino acid sequence of CXCL14 fragment 2
Fig. 23: Fig. 23 shows the amino acid sequence of CXCL14 fragment 3
Fig. 24: Fig. 24 shows the amino acid sequence of CXCL14 fragment 4
Fig. 25: Fig. 25 shows the amino acid sequence of CXCL14 fragment 5
Fig. 26: Fig. 26 shows the amino acid sequence of CXCL14 fragment 6
Fig. 27: Fig. 27 shows the amino acid sequence of CXCL14 fragment 7
Fig. 28: Fig. 28 shows the amino acid sequence of CXCL14 fragment 8
Fig. 29: Fig. 29 shows the amino acid sequence of CXCL14 fragment 9
Fig. 30: Fig. 30 shows the amino acid sequence of CXCL14 fragment 10
Fig. 31: Fig. 31 shows the amino acid sequence of CXCL14 fragment 11
Fig. 32: Fig. 31 shows the amino acid sequence of CXCL14 fragment 12
Fig. 33: Fig. 33 shows the activity of different CXCL14 peptide fragments (1 to 7) on MrgX2 in a cell based assay format (Fluo8 assay) (RFU: relative fluorescence units). A. CXCL14 and PAMP; B. Fragment 1, 2, 5; C. Fragment 3, 4, 6, 7

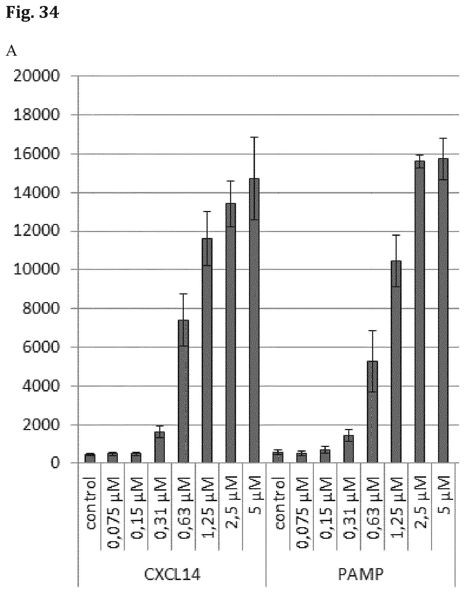

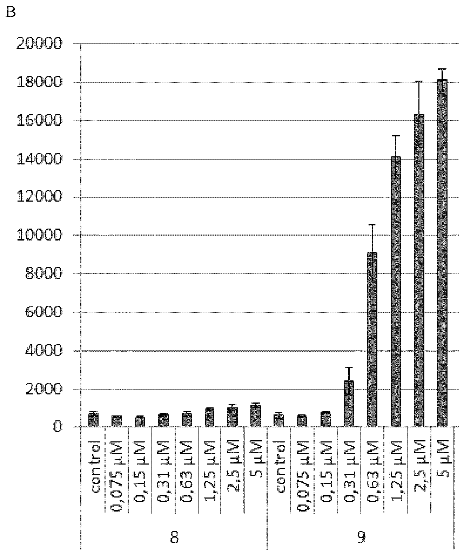

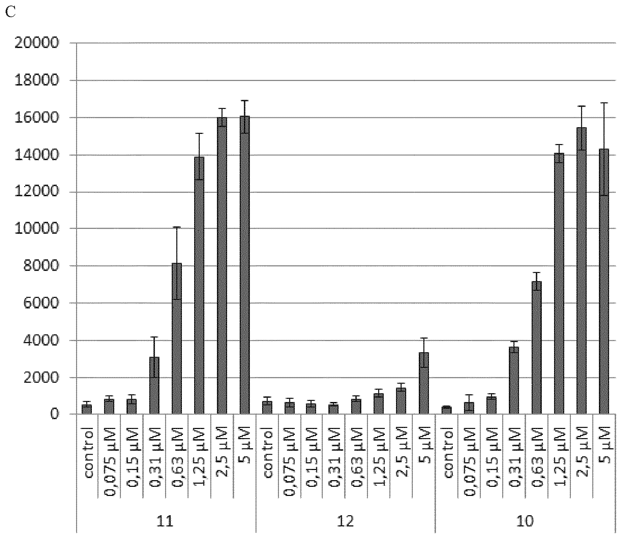

Fig. 34: Fig. 34 shows the activity of different CXCL14 peptide fragments (8 to 12) on MrgX2 in a cell based assay format (Fluo8 assay) (RFU: relative fluorescence units). A. CXCL14 and PAMP; B. Fragment 8, 9; C. Fragment 11, 12, 10

### Detailed description of the invention

### Identification of CXCL14 receptor

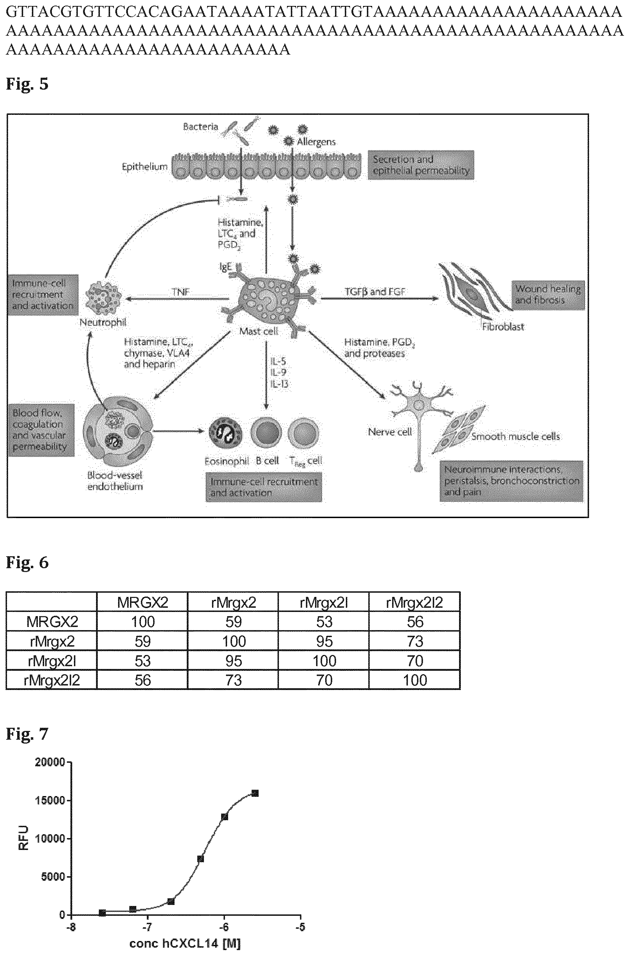

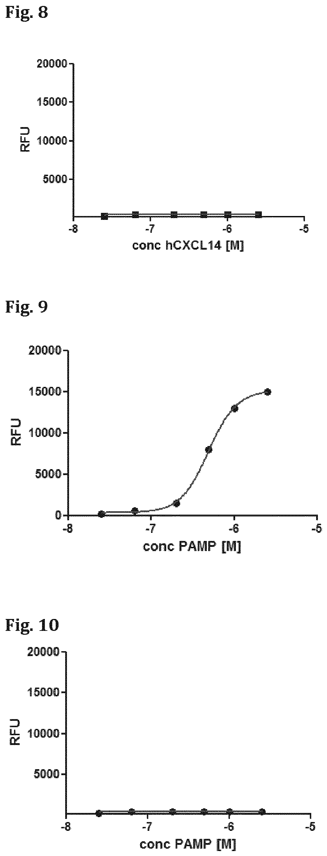

The MrgX2 receptor was tested for CXCL14 dependent interacellular calcium release. Surprisingly MrgX2 was found to be activated by CXCL14 in a dose dependent matter. A dose response curve for CXCL14 activating MrgX2 in a cell based assay format is shown in fig. 7. The used cell line is overexpressing MrgX2 in CHEM1 by recombinant expression. CXCL14 shows no significant calcium release on CHEM1 cell without MrgX2 overexpression (as shown in fig. 8) under the same experimental conditions. The EC50 of human CXCL14 on CHEM1-MrgX2 was calculated as 5.7x10⁻⁰⁷ M.
Mouse CXCL14 shows a dose dependent Calcium release on CHEM1-MrgX2 (but not on CHEM1 without MrgX2) with a comparable EC50.

The known MrgX2 agonist PAMP shows a dose dependent Calcium release in CHEM1-MrgX2 , but not in CHEM1 (without MrgX2) cell line (as shown in fig. 9 for PAMP on CHEM1-MrgX2 and fig. 10 for PAMP on CHEM1). The EC50 of PAMP on CHEM1-MrgX2 was calculated as 4.9x10⁻⁰⁷ M. Furthermore we found that CXCL14 is an antagonist of the G-protein coupled receptor CXCR4.

### Specificity of CXCL14

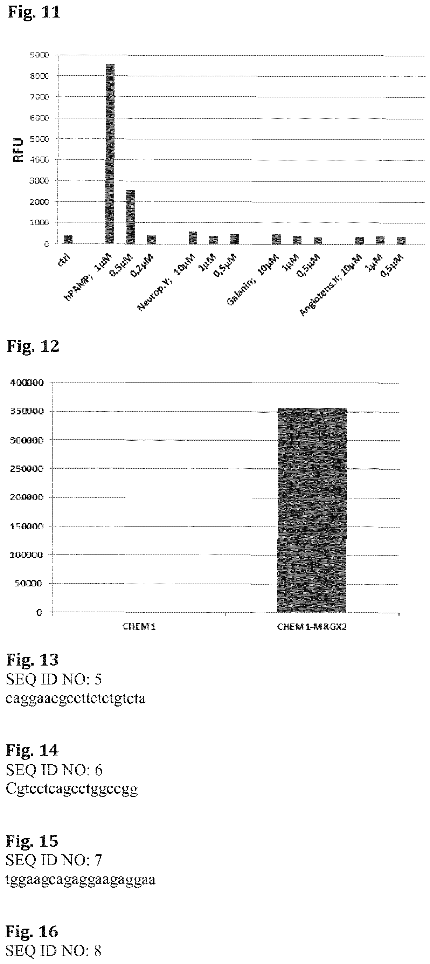

To characterize the specificity of CXCL14 to MrgX2 activation we have analyzed 3 peptides (NPY, angiotensin II and galanin) in a cell based Calcium release assay with CHEM1-MrgX2 and CHEM1 cell lines. Neither NPY or angiotension II nor galanin show a dose dependent Calcium release on both tested cell lines (as shown in fig. 11). We could show that CXCL14 is a specific agonist of MrgX2.

To confirm the overexpression of MrgX2 in CHEM1-MrgX2 cells only, we have performed a QPCR analysis of total RNA samples from both cell lines. As shown in fig. 12 only in CHEM1-MrgX2 total RNA we could detect human MrgX2 RNA. In CHEM1 (control) MrgX2 was not detectable. We could show that CXCL14 is a specific agonist of MrgX2.

### Identification of CXCL14 derived peptides as modulators of MrgX2 and CXCL14 activity

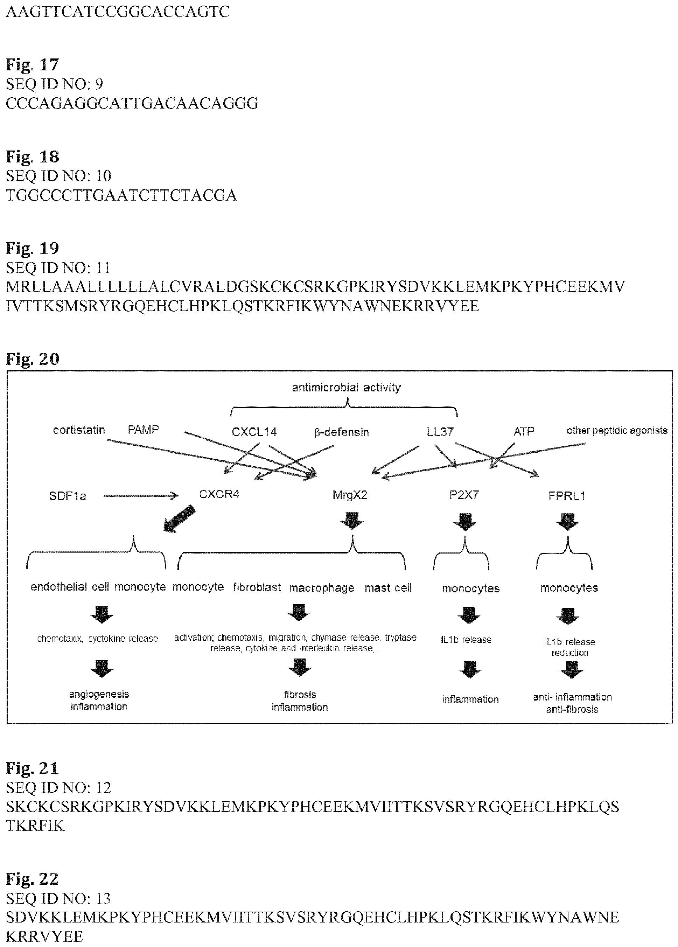

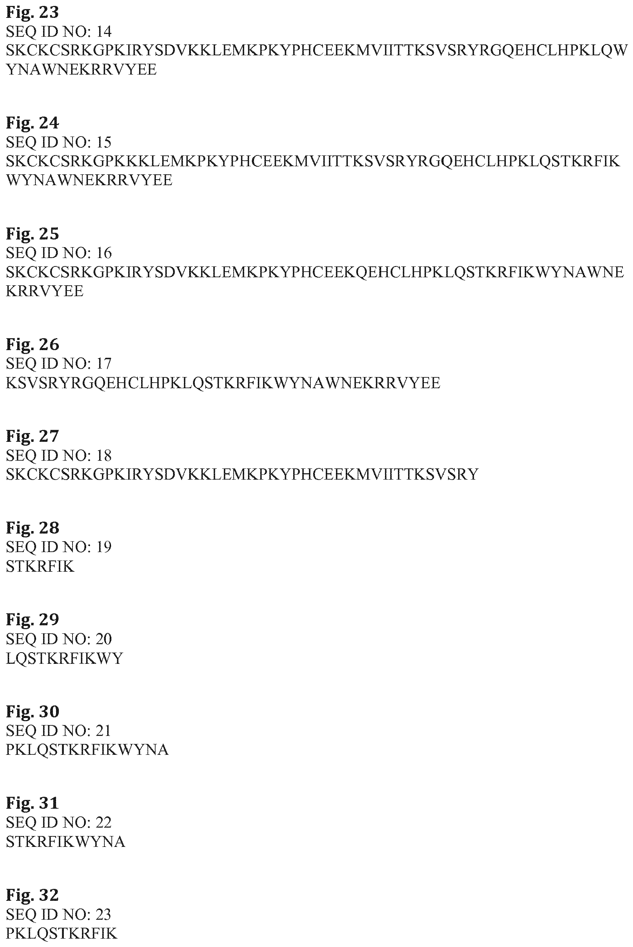

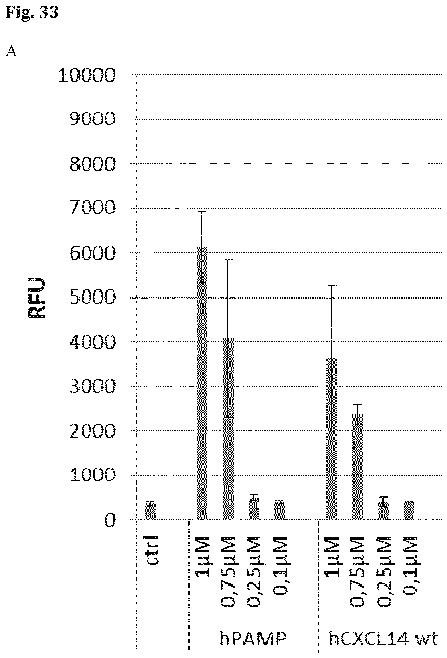

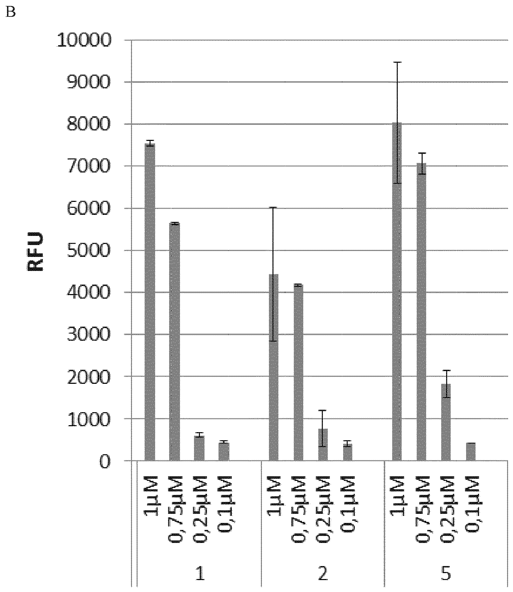

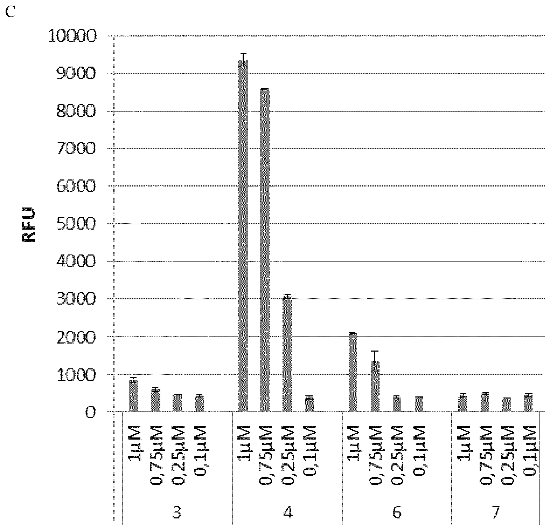

To identify CXCL14 derived peptides with modulated CXCL14 activity, different peptides were designed, synthetized and characterized. The sequences of CXCL14 derived peptides 1 to 12 are given in fig. 22 to 32. All peptides were tested on CHEM1-MrgX2 and CHEM1 (control) cell lines using a Fluo8 based readout (as in example 2). Surprisingly truncated peptides with defined sequences show CXCL14 activity on MrgX2. Peptides 12 and 8 show low activity only (as shown in fig 33).

Truncated CXCL14 based peptides have different characteristics regarding pharmacokinetic, activity, solubility or other important properties for pharmacological treatment or drug development. The described peptides or variants thereof could be useful for the treatment of CXCL14, MrgX2 or CXCR4 related diseases as cancer, pulmonary fibrosis, infection and bactericidal activity, kidney disease, macula disease, colitis, angiogenesis, arthritis, diabetes (glucose metabolism), asthma, COPD, pulmonary embolism, cns disorders and diseases associated with the modulation of the SDF1a-CXCR4 pathway, mast cell activity, dorsal root ganglia function or inflammation.

As CXCL14 is described as an inhibitor of the SDF1-CXCR4 pathway [40] the disclosed CXCL14 related peptides and variants thereof could be used as modulators of the pathway.

A further embodiment of the disclosure is a CXCL14 related peptide or variant thereof comprised in the group of fragments consisting of fragment 1, 2, 4, 5, 9, 10, and 11.
A preferred embodiment of the disclosure is peptide PKLQSTKRFIKWYNA (SEQ ID NO: 21) or a variant thereof. A further preferred embodiment of the disclosure is peptide, , LQSTKRFIKWY (SEQ ID NO: 20) or a variant thereof. A further preferred embodiment of the disclosure is peptide and STKRFIKWYNA (SEQ ID NO: 22) or a variant thereof. The aforementioned peptides or variants thereof are useful for the characterization and analysis the CXCL14- or SDF1- or MrgX2 pathways e.g. in screening assays and the development of modulators of those pathways and as modulator for the SDF1/CXCR4 pathway.

### MrgX2 (MRGPRX2)

G protein coupled receptors (GPCRs), also known as seven-transmembrane domain receptors, 7TM receptors, heptahelical receptors, serpentine receptor, and G protein-linked receptors (GPLR), comprise a large protein family of transmembrane receptors that sense molecules outside the cell and activate inside signal transduction pathways and, ultimately, cellular responses. The ligands that bind and activate these receptors include light-sensitive compounds, odors, pheromones, hormones, and neurotransmitters, and vary in size from small molecules to peptides to large proteins. G protein-coupled receptors are involved in many diseases, and are also the target of approximately 40% of all modem medicinal drugs [8].

There are two principal signal transduction pathways involving the G protein-coupled receptors: the cAMP signal pathway and the phosphatidylinositol signal pathway. When a ligand binds to the GPCR it causes a conformational change in the GPCR, which allows it to act as a guanine nucleotide exchange factor (GEF). The GPCR can then activate an associated G-protein by exchanging its bound GDP for a GTP. The G-protein's α subunit, together with the bound GTP, can then dissociate from the β and y subunits to further affect intracellular signaling proteins or target functional proteins directly depending on the α subunit type (Gαs, Gαi/o, Gαq/11, Gα12/13) [9].

Recently, a large family of G-protein-coupled receptors called Mas-related genes (Mrgs), which is selectively expressed in small-diameter sensory neurons of dorsal root ganglia, was described. A subgroup of human Mrg receptors was defined as MrgX1-X4. MrgX receptors were cloned from rhesus monkey and functionally characterized alongside their human orthologs. Most of the human and rhesus MrgX receptors displayed high constitutive activity in a cellular proliferation assay. Proliferative responses mediated by human or rhesus MrgX1, or rhesus MrgX2 were partially blocked by pertussis toxin (PTX). Proliferative responses mediated by rhesus MrgX3 and both human and rhesus MrgX4 were PTX insensitive. These results indicate that human and rhesus MrgX1 and MrgX2 receptors activate both Gq- and Gi-regulated pathways, while MrgX3 and MrgX4 receptors primarily stimulate Gq-regulated pathways. Peptides known to activate human MrgX1 and MrgX2 receptors activated the corresponding rhesus receptors in cellular proliferation assays, Ca(2+)-mobilization assays, and GTP-gammaS-binding assays. Cortistatin-14 was selective for human and rhesus MrgX2 receptors over human and rhesus MrgX1 receptors. BAM22 and related peptides strongly activated human MrgX1 receptors, but weakly activated rhesus MrgX1, human MrgX2, and rhesus MrgX2 receptors. These data suggest that the rhesus monkey may be a suitable animal model for exploring the physiological roles of the MrgX receptors [30]. MrgX2 serves as a receptor for the antimicrobial peptide LL37 (CAMP) [29]. It is reported that a human mast cell line expressing MRGX2, as well as mast cells derived from pluripotent stem cells, responded to LL37 (CAMP) for sustained Ca(2+) mobilization and degranulation. In contrast, an immature mast cell line lacking MRGX2 and a mast cell line treated with short hairpin RNA (shRNA) against MRGX2 did not respond to LL37. Mast cell lines stably expressing MRGX2 responded to LL37 by chemotaxis, degranulation, and CCL4 production. MRGX2 resisted LL37-induced phosphorylation, desensitization, and internalization.

The sequences of human MrgX2 are given in fig.s 3 and 4 (amino acid and nucleotide sequence respectively; SEQ ID NO: 3 and 4).

### Diseases associated with MrgX2

MrgX2 is described in literature to be possibly associated with diseases or disorders of (but not limited to): infections [29], inflammation [29, 31], mast cell related diseases [29], immune system [32], cancer [33] and nociception / pain [34].

### MrgX2 ligands and modulators

Known MrgX2 agonistic modulatorsareLL37 [22]), PAMP [38], Corticostatin 14 [35, 36], BAM22 [35], and Morphine [37]. Furthermore several peptidic and non-peptidic modulators are described [35].

Additionally, Cortistatin 17, Somatostatin (different isoforms), NPFF, Oxytocin, Cyclosomatostatin, Dynorphin A, [Arg8] vasopressin, Substance P, and Hexarelin [82] are described as MrgX2 modulator.

### Mast cells

A mast cell (also known as mastocyte and labrocyte) is a resident cell of several types of tissues and contains many granules rich in histamine and heparin. Although best known for their role in allergy, inflammation and anaphylaxis, mast cells play an important protective role as well, being intimately involved in wound healing and defense against pathogens. The mast cell is very similar in both appearance and function to the basophil, a type of white blood cell. However, they are not the same, as they both arise from different cell lines.

Mast cells (MC) have the uniqueness of being tissue resident cells packaged with cytoplasmic granules full of preformed mediators of diverse nature, rich in surface receptors that upon ligand binding can induce not only the fast release of the stored mediators, but also the de novo synthesis of arachidonic acid metabolites and a number of chemokines and cytokines. Therefore they play a prominent role in maintaining homeostasis, acting as armed sentinel cells in the tissues, where they reside spanning from mucosal to connective tissues and more. However, their strategic location and potential have clearly demonstrated that MCs are more than inflammation; they are very important players also in innate and adaptive immune responses, inflammation, and tissue changes. MC immunomodulatory roles may result in either negative or positive outcome for the host, enhancing, or suppressing certain features of immune/inflammatory responses [7]. The effect of mast cell activation within the inflammation, immune pathways and initiation of tissue remodelling is shown in fig. 5.

### Diseases associated with mast cells

Diseases or disorders described as to be involved with mast cells and mast cell activation, but not limited to:
Immunological/neoplastic diseases: Carcinoid tumour, Pheochromocytoma, Primary gastrointestinal allergy, Hypereosinophilic syndrome , Hereditary angioedema, Vasculitis, Intestinal lymphoma [39]
Gastrointestinal disorders: Helicobacter-positive gastritis, Infectious enteritis, Eosinophilic gastroenteritis, Parasitic infections, Inflammatory bowel disease, Celiac disease, Primary lactose intolerance, Microscopic colitis, Amyloidosis, Intestinal obstructions by adhesions, volvulus and other reasons, Hepatitis, Cholelithiasis, Hereditary hyperbilirubinemia [39]
Endocrinologic disorders: Diabetes mellitus, pancreatic endocrine tumours, porphyria, disorders of the thyroid gland, Morbus Fabry [39]

### Mastocytosis [44]

### Mast Cell Activation Disorder (MCAD) or syndrome [45, 51]

Other: vascular leakage [46], cardiomyopathy [47], idiopathic pulmonary fibrosis [48], mucosal disease [49], vascular remodelling [50], pulmonary arterial hypertension [52],pulmonary hypertension [58], drug allergy [53], autoimmune disease [54], renal fibrosis [55], interstitial cystitis [56], COPD [57], asthma [60], anaphylaxis [61], myocarditis [62], aortic aneurysm formation [63], sarcoidosis [64], cystic fibrosis [65], renal diseases [66], immunological skin diseases [67], myocardial infarction [68], acute coronary syndromes [69], hepatic fibrosis [70], multiple sclerosis [71], acute and chronic arthritis [72], cardiac fibrosis [73], heart failure [74], cardiomyopathy [75], vascular diseases [76], cancer [77], ophthalmology [78] and others.

### MrgX2 in dorsal root ganglions

MrgX2 is described as expressed in dorsal root ganglions [81]. A dorsal root ganglion (or spinal ganglion) is a nodule on a dorsal root of the spine that contains cell bodies of nerve cells (neurons) that carry signals from sensory organs toward the appropriate integration center. Nerves that carry signals toward the central nervous system (brain or spinal cord) are known as afferent nerves. Dorsal root ganglions are involved in somatosensory transduction, mechanosensitive and nociception. The modulation of CXCL14 mediated modulation of MrgX2 activity should have effects of the dorsal root ganglion functions. The inhibition of CXCL14 dependent MrgX2 activation should be useful for the treatment of nociception and sensory transduction related disease.

### Drug discovery

The modern drug research process has reversed the classical pharmacological strategy. Today, research programs are initiated based on biological evidence suggesting a particular gene or gene product to be a meaningful target for small molecule drugs useful for therapy. The process envisioned to identify target-specific modulators lacking several side-effects. Also, it allows setting up a linear drug discovery process starting from target identification to finally delivering molecules for clinical development. One central element is lead discovery through High-Throughput screening of comprehensive corporate compound collections. Pharma research in most organizations is organized in discrete phases together building a "value chain" along which discovery programs process to finally drug candidated for clinical testing.

Today a large arsenal of technologies is available for researchers in industry and academia to generate data in support of a functional link between a given gene and a disease state. Big pharmaceutical companies typically have established specialized groups dedicated to target biology. Generation of a hypothetical diseases linkage frequently is followed by a series of experiments designed to further validate the initial hypothesis. In many cases, high-throughput technologies like Real-Time PCR using TaqMan detection systems have been implemented to systematically screen for genes with interesting expression patterns or significant regulation in relevant disease state. As a result, most companies have established comprehensive bioinformatic databases containing molecular biology and expression data. Such data are further supplemented by information derived from generic and population studies and transgenic animals. The introduction of RNAi technology marks another technologcal advancemant employed for target identification and validation in vitro and in laboratory animals. Following the sequencing and publication of the human genome in 2001 the catalogue of potential target candidates is publically available. However, our understanding of gene function and their potential relevance for the treatment of diseases still remains obscure.

Following a technical assesment of the targets "drugability" [1], the probability to identify small molecule modulators, and technical feasibility target-specific assays are developed to probe the corporate compound collection for meanful leads. Lead discovery in the pharmaceutical industry today still depends largely on experimental scrrening of compound collections. To this end, the industry has invested heavily in expanding theur compound files and established appropiate screening capabilities to handle large numbers of compounds within a reasonable period of time. "High-Troughput-Screening" (HTS) started roughly one decade ago with th introduction of laboratory automation to handle the different assay steps typically performed in microtiter plates. HTS technologies during the last decade have witnessed remarkable developments. Assay technologies have advanced to provide a large variety of various cell-based and biochemical test formats for a large spectrum of disease relevant target classes [2]. In parallel, further miniaturization of assays volumes and parallelization of processing have further increased the test throughput. For example, HTS can be performed entirely in 1536-well plates with assay volumes between 5-10 µl. This assay carrier together with fully-automated robotic systems allow for testing in excess of 200,000 compounds per day. Such Ultra-high-throughput is required to exploit compound files of >1.5million compounds. Such comprehensive substance collections, together with sophisticated screening technologies have resulted in a clear advantage in lead discovery especially for poorly druggable targets with a poor track record in the past. In addition, size and composition of the library with compounds largely proprietary to the company facilitates the identification of leads providing a straight forward path towards establishing intellectual property on composition of matter [3]. Effort sto identify antibodies are routinely performed e.g. by phage display technologies.

### Test Compounds

Suitable test compounds for use in the screening assays of the invention can be obtained from any suitable source, e.g., conventional compound libraries. The test compounds can also be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds [Lam, (1997)]. Examples of methods for the synthesis of molecular libraries can be found in the art. Libraries of compounds may be presented in solution or on beads, bacteria, spores, plasmids or phage.

### Binding Assays

For binding assays, the test compound is preferably a small molecule which binds to and occupies the active site of a Mrg receptor polypeptide, thereby making the CXCL14 binding site inaccessible to substrate such that normal biological activity is prevented. Examples of such small molecules include, but are not limited to, small peptides or peptide-like molecules. In binding assays, either the test compound, the Mrg receptor or the CXCL14 polypeptide can comprise a detectable label, such as a fluorescent, radioisotopic, chemiluminescent, or enzymatic label, such as horseradish peroxidase, alkaline phosphatase, or luciferase. Detection of a test compound which is bound to the Mrg receptor can then be accomplished, for example, by direct counting of radioemmission, by scintillation counting, or by determining conversion of an appropriate substrate to a detectable product. Alternatively, binding of CXCL14 to the Mrg receptor polypeptide can be determined without labeling either of the interactants. For example, a microphysiometer can be used to detect binding of a CXCL14 polypeptide with a Mrg receptor in the presence or absence of a test compound. A microphysiometer (e.g., Cytosensor™) is an analytical instrument that measures the rate at which a cell acidifies its environment using a light-addressable potentiometric sensor (LAPS). Changes in this acidification rate can be used as an indicator of the interaction between CXCL14 polypeptide with a the Mrg receptor in the presence or absence of a test compound [Haseloff, (1988)].
Determining the ability of a CXCL14 polypeptide with a Mrg receptor in the presence or absence of a test compound also can be accomplished using a technology such as real-time Bimolecular Interaction Analysis (BIA) [McConnell, (1992); Sjolander, (1991)]. BIA is a technology for studying biospecific interactions in real time, without labeling any of the interactants (e.g., BIAcoreTM). Changes in the optical phenomenon surface plasmon resonance (SPR) can be used as an indication of real-time reactions between biological molecules.

Agonists of a Mrg receptor are molecules which, when bound to a Mrg receptor, increase or prolong the activity of a Mrg receptor. Agonists of a Mrg receptor include proteins, nucleic acids, carbohydrates, small molecules, or any other molecule which activate FAP. Antagonists of a Mrg receptor are molecules which, when bound to a Mrg receptor, decrease the amount or the duration of the activity of a Mrg receptor stimulated by a ligand, preferably a CXCL14 polypeptide. Antagonists include proteins, nucleic acids, carbohydrates, antibodies, small molecules, or any other molecule which decrease the activity of a Mrg receptor. A preferred antagonist of the disclosure is an antagonist of human MrgX2 activation by human CXCL14 polypeptide. An even more preferred antagonist is said antagonist which is not an antagonist of human MrgX2 activation by another MrgX2 agonist. This allows to specifically interfere with the human CXCL14 polypeptide mediated activation of human MrgX2. Preferably, said antagonist does not interfere with LL37, PAMP, Corticostatin 14, BAM22, or Morphine mediated activation of MrgX2.
This invention is particularly useful for screening therapeutic compounds by using MrgX2 and CXCL14 in any of a variety of drug screening techniques. As MrgX2 is a G protein coupled receptor any of the methods commonly used in the art may potentially be used to identify MrgX2 modulators. For example, the activity of a G protein coupled receptor such as MrgX2 can be measured using any of a variety of appropriate functional assays in which activation of the receptor results in an observable change in the level of some second messenger system, such as adenylate cyclase, guanylylcyclase, calcium mobilization, or inositol phospholipid hydrolysis. Alternatively, the polypeptide or fragment employed in such a test is either free in solution, affixed to a solid support, borne on a cell surface or located intracellularly. One method of drug screening utilizes eukaryotic or prokaryotic host cells which are stably transformed with recombinant nucleic acids expressing the polypeptide or fragment. Drugs are screened against such transformed cells in competitive binding assays. Such cells, either in viable or fixed form, are used for standard binding assays. Measured, for example, is the formation of complexes between MrgX2 and CXCL14 dependent on the agent being tested. Alternatively, one examines the diminution in complex formation between MrgX2 and CXCL14 dependent on the agent being tested.

### Species differences

CXCL14 (originally identified as BRAK, BMAC or Mip-2a) was isolated as a gene whose expression is lost or down-regulated in various human cancer cell lines and tumour specimens. The 99 amino acid residue precursor of CXCL14 has a 22 amino acid signal peptide that is cleaved to produce a 77 amino acid mature protein. The calculated molecular weight of the human CXCL14 protein is 9.4 kDa with an isoelectric point of 10.3. Human and murine CXCL14 differ by only two amino acid residues. The amino acid composition of CXCL14 is well conserved between humans, birds, frogs and fish [22, 23].

A large family of G-protein-coupled receptors called Mas-related genes (Mrgs) are described in different species. The number of receptors described for each subfamily is species dependent. Especially in mouse and rat a larger number of MrgX family members are described, but not well characterized.

### Chemokines in mast cells

In addition to their role as sentinels in the recognition of pathogens, mast cells (like platelets) are able to communicate with immune cells facilitating the recruitment of leukocytes to sites of infection. Indeed, mast cells are able to produce different chemokines including CCL4, CXCL8, or CCL11 assisting in the recruitment of CD8+ T cells, eosinophils, and natural killer cells, respectively [5]. CCL3/MIP-1a and CCL4/MIP-1b can initiate diverse cellular responses that regulate both acute and chronic inflammation via their interaction with CCR1 and CCR5. In addition, proteoglycan-bound CCL4 is used to effectively activate and induce the adhesion of circulating lymphocytes for their extravasation through lymph node endothelium. The quaternary structures of CCL3 and CCL4 are decisive for their biological activity. The continuous and slow release of monomers from the polymer leads to a shallow gradient with a long gradient and effective range for leukocyte recruitment. CXCL8/interleukin-8/IL8 has been found in intracellular granules from skin mast cells and mast cell lines [6]. Recently it is known that CXCL8 synthesis is induced via the leukotriene B4/leukotriene B4 Receptor 2 pathway in response to IL-1b in human primary mast cells and mast cell line HMC-1. CXCL8 released by mast cells is implicated in the selective chemotaxis of CXCR1-expressing natural killer cells. CXCL8 also induces neutrophil migration and activation by binding to G-protein-coupled receptors on their surface, namely human CXCR1 and CXCR2. During inflammation, CXCL8 is produced and presented to the endothelial surface in association with GAGs. Also, this chemokine is among the most important in the recruitment of inflammatory cells, mostly neutrophils, in COPD and lung disease [4]. So far the impact or function of CXCL14 on mast cell or mast cell activation is not described.

### Mast cell activation

For the determination of mast cell activation different methods are described in the literature [59]. Most methods are based on the determination of released protein levels or transcriptional changes.

### CXCL14 network and pathway associations

CXCL14 is part of a receptor network which is involved in different physiologically processes. Apart from the antimicrobial activity of CXCL14, the effect of CXCL14 on cell activation is mediated by the modulation of MrgX2 activity. In fig. 20 the links of CXCL14 to its receptor MrgX2 and selected receptors, ligands, cells and its disease associations are shown.

Surprisingly we have identified MrgX2 as the receptor of CXCL14. The modulation of MrgX2 activity by inhibition of CXCL14 dependent receptor activation or binding could lead to a modulation of other known MrgX2 agonists or ligands dependent receptor activation. The inhibition of the CXCL14 dependent MrgX2 activity should lead to the modulation of the MrgX2 mediated cell activation of mast cells, macrophages, monocytes or fibroblasts (but not limited to). The role of the activation of those cell lines in different diseases is described in the literature.

The inhibition of the CXCL14 dependent MrgX2 activity should be reasonable for the treatment of those diseases. The inhibition of the CXCL14 dependent MrgX2 activity by the use of receptor modulators, keep the antimicrobial activity of CXC14 and other MrgX2 ligands (as LL37) untouched. The maintenance of the CXCL14/LL37/defensin antimicrobial pathways should create an additional positive effect within disease treatment.

Disclosed here are:
1. A method of screening for an antagonist of a Mrg receptor comprising the steps of
   a. contacting a test compound with a Mrg receptor polypeptide in the presence of a CXCL14 polypeptide,
   b. detecting binding of a CXCL14 polypeptide to said Mrg receptor polypeptide in the presence of said test compound,
   c. contacting CXCL14 with a Mrg receptor polypeptide in the absence of said test compound, and
   d. detecting binding of a CXCL14 polypeptide to said Mrg receptor polypeptide in the absence of said test compound. In an even more preferred embodiment the method comprises a further step e., wherein an antagonist is determined based on the difference of CXCL14 polypeptide binding of step b. and d. An antagonist is determined when the binding of a CXCL14 polypeptide of step b. is lower than in step d.
2. A method of screening for an antagonist of a Mrg receptor comprising the steps of
   a. determining the activity of a Mrg receptor polypeptide in the presence of a CXCL14 polypeptide, and
   b. determining the activity of a Mrg receptor polypeptide in the presence of a CXCL14 polypeptide and a test compound. In an even more preferred embodiment the method comprises a further step c., wherein an antagonist is determined based on the difference of Mrg receptor activity of step a. and b. An antagonist is determined when the Mrg receptor activity of step b. is lower than in step a.
3. The method of any one of the preceding embodiments, wherein additionally the binding or activity of an MrgX2 agonist is determined in the presence or absence of said test compound is determined.
4. A method according to embodiment 3, wherein the MrgX2 agonist is comprised in the group of agonists consisting of LL37, PAMP, BAM22, Cortistatin 17, Somatostatin, Somatostatin isoforms, NPFF, Oxytocin, Cyclosomatostatin, Dynorphin A, [Arg8] vasopressin, Substance P and Hexarelin.
5. A method of screening for an agonist of a Mrg receptor comprising the steps of
   a. generating a CXCL14 variant by replacing one or more amino acids of a CXCL14 polypeptide by another naturally or non-naturally occurring amino acid, and
   b. determining the activity of a Mrg receptor polypeptide in the presence of a CXCL14 variant.
6. The method of any one of the preceding embodiments, wherein the Mrg receptor is MrgX2.
7. The method of any one of the preceding embodiments, wherein the Mrg receptor is human MrgX2. In a more preferred embodiment human MrgX2 is depicted in SEQ ID NO: 3. In another embodiment human MrgX2 can vary at one or more amino acid position from SEQ ID NO: 3 provided that the molecule has MrgX2 activity.
8. The method of any one of the preceding embodiments, wherein CXCL14 polypeptide is human CXCL14 polypeptide. In a more preferred embodiment human CXCL14 is depicted in SEQ ID NO: 1. In another embodiment human CXCL14 can vary at one or more amino acid position from SEQ ID NO: 1 provided that the molecule has CXCL14 activity, e.g. a CXCL14 variant.The method of any one of the preceding embodiments, wherein the step of contacting is in or at the surface of a cell.
9. The method of any one of the preceding embodiments, wherein the cell is in vitro.
10. The method of any one of the preceding embodiments, wherein the step of contacting is in a cell-free system.
11. The method of any one of the preceding embodiments, wherein the CXCL14 polypeptide or CXCL14 variant is coupled to a detectable label.
12. The method of any one of the preceding embodiments, wherein the compound is coupled to a detectable label.
13. The method of any one of the preceding embodiments, wherein the CXCL14 polypeptide or CXCL14 variant is attached to a solid support.
14. The method of any one of the preceding embodiments, wherein the compound is attached to a solid support.

### Examples

### Example 1

### Expression analysis (QPCR)

For relative quantitation of MrgX2 mRNA levels in Chem1 cells, a TaqMan™ real-time PCR assay was employed on a LC480 (Roche, Mannheim, Germany) according to the manufacturer's protocols. cDNA was performed according to the manufacturers protocol with Omniscript reverse transcriptase (Qiagen, Hilden, Germany) in the supplied buffer plus 9.5 µM random hexamer primer, 0.5 mM per dNTP, and RNaseOUT™ (Invitrogen). A PCR reaction mix (25 µl) contained, in addition to cDNA, 0.2 µM per amplification primer and 0.2 µM FAM™/TAMRA™-labelled probe and TaqMan reaction mix. The thermal protocol was set to 2 min at 50 °C, followed by 10 min at 95 °C, followed by 40 cycles of 15 s at 95 °C and 1 min at 60 °C. To normalize the amount of cDNA per assay, the expression of L32 was measured in parallel. Relative expression of MrgX2 was then calculated using the normalized expression values to L32 (human RL32).

### Example 2

### Determination of mast cell activation

To determine the β-hexosaminidase activity in MC/9 cells or mast cells, cells were degranulated in HEPES-tyrode supplemented with 0.1 % fatty acid free BSA. 50 µL of supernatant was added to 50 µL 2 mM 4-nitrophenyl N-acetyl-b-D-glucosaminide in 0.2 M citrate (pH 4.5) and incubated at 37 °C for 2 hours. After addition of 150 µL 1 M Tris (pH 9.0), absorbance was measured at 405 nm. To measure chymase and tryptase release after degranulation, 50 µL supernatant was added to 2 mM S-2288 (tryptase substrate, Chromogenix, Lexington, USA) or S-2586 (chymase substrate, Chromogenix) in PBS supplemented with 100 U/mL heparin. After 2 hours (tryptase) or 48 hours (chymase) at 37 °C, OD405 was measured. Histamine and VEGF levels were measured with a Histamine ELISA (Neogen, Lansing, MI) and a VEGF ELISA (Biosource, Etten-Leur, The Netherlands) according to manufactuers instructions. 2.5*10E5 MC/9 cells or mast cells were lysed in 1 mL 2% Triton in HEPES-tyrode and used for total release in each assay and supernatant of non-degranulated mast cells was used as control [81].

### Example 3

### Fluo8 assay

The Fluo8 assay is a cell based assay format for the determination of intracellular Calcium release. Cell were seeded on microtiter plates and incubated under standard cell culture condtions. After 24h the medium was removed and the cells were washed with PBS. The cells were incubated with loading buffer (40 ml PBS with 2mM Calcium + 800 µl brilliant black (2%) + 732 µl pobenecid (0,5 M) in 1N NaOH + 400 µl Pluronic (10%) in water + 152 µl Fluo8 (5,7 µg/ml) in water) for 30 minutes. For fluorescence readout and compound testing a FLIPR tetra system was used according to the manufactures protocol (Molecular Devices, CA 94089-1136, USA).

### Example 3

### Cell culture

The used cell lines CHEM1 and CHEM1-MrgX2 are adherent rat cell lines (Millipore, Billerica, MA 01821, USA) with were cultures under standard cell culture conditions.

### Example 4

### CXCR4 activity assay

The CXCR4 receptor is a chemokine receptor and belongs to the family of G protein coupled receptors (GPCRs). It's only known endogenous ligand is stromal cell-derived factor 1 (SDF-la). CXCR4 is a Gi coupled receptor. Its activation leads to a decrease of intracellular cAMP, which could be monitored by recombinant cAMP-responsive element (CRE) regulated luciferase expression. For the characterization of modulators of CXCR4 activity, stable cell lines expressing recombinant CXCR4 and a CRE-luciferase reporter vector were established. The cells were incubated with forskolin at a concentration of 0.5 µM, SDF-1a (at 20 nM) and test compounds and incubated for 4h under standard cell culture conditions. Luciferase substrate was added and the luminescence was measured for 60 seconds.

### References

1. Hopkins & Groom, Nature Drug Discovery 2002
2. Walters, W.P. et al. (2003) Nature Reviews in Drug Discovery 2, 259-266
3. Golz, S. and Hüser, J. (2007) Clin. Lab. 53:77-79
4. von Hundelshausen et al (2012) Front Immunol. 3:175
5. Abraham,S.N. et al. (2010) Nat.Rev. Immunol. 10, 440-452.
6. Möller,A. et al. (1993) J. Immunol. 151, 3261-3266.
7. Schaffer et al. (2012) Front Immunol. 3:238
8. Filmore, David (2004) Modern Drug Discovery (American Chemical Society) 24-28
9. Kolakowski LF Jr (1994) Receptors Channels 2 (1): 1-7
10. Hromas, R. et al. (1999) Biochem. Biophys. Res. Commun. 255: 703-706
11. Shellenberger TD et al. (2004) Cancer Res. 2004 Nov 15;64(22):8262-70
12. Tessema M. et al. (2010) Oncogene. 2010 Sep 16;29(37):5159-70
13. Emblom-Callahan MC et al. (2010) Genomics. 2010 Sep;96(3):134-45
14. Maerki C et al. (2009) J Immunol. 2009 Jan 1;182(1):507-14.
15. Kleer CG et al. (2008) Clin Cancer Res. 2008 Sep 1;14(17):5357-67.
16. Bennett MR et al. (2007) Nephron Exp Nephrol. 2007;107(1):e30-40.
17. Radeke MJ et al. (2007) Exp Eye Res. 2007 Sep;85(3):366-80.
18. Abad C et al. (2005) Inflamm Bowel Dis. 2005 Jul;11(7):674-84.
19. Hromas R et al. (1999) Biochem Biophys Res Commun. 1999 Feb 24;255(3):703-6.
20. Ferreira MA et al. (2011) Eur J Hum Genet. 2011 Apr;19(4):458-64.
21. Beaven et al. (2011) Crit Rev Immunol. 31(6): 475-529.
22. Hara, et al. (2012) J. Biochem. 151(5):469-76
23. Huising, M.O. et al. (2004) Eur. J. Biochem. 271, 4094-4106
24. Kurth, I. et al. (2001) J. Exp. Med. 194, 855861
25. Schaerli, P et al. (2005) Immunity. 23, 331342
26. Shellenberger, T.D et al. (2004) Cancer Res. 64, 82628270
27. Shurin, G.V., Ferris, R. et al. (2005) J. Immunol. 174, 54905498
28. Chen, L. et al. (2010) J. Immunol. 184, 4455-4459
29. Subramanian, H. et al. (2011) J. Biol. Chem. 286: 44739-44749
30. Burstein eta al. (2006) Br J Pharmacol. Jan;147(1):73-82.
31. Kashem et al. (2011) Eur J Pharmacol. Oct 1;668(1-2):299-304.
32. Von Hagen et al. (2008) Mol Cell Endocrinol. May 14;286(1-2):141-7.
33. Volante et al. (2008) Mol Cell Endocrinol. May 14;286(1-2):219-29.
34. Yang S et al. (2005) Gene. Jun 6;352:30-5. Epub 2005 Apr 26.
35. Malik et al. (2009) Bioorganic & Medicinal Chemistry Letters 19 1729-1732
36. Robas et al. (2003) J Biol Chem. Nov 7;278(45):44400-4.
37. Natsuyo et al. (2007) Journal of Cell and Animal Biology Vol. 2 (1), pp. 004-009,
38. Katou et al. (2005) Biochem Biophys Res Commun. May 20;330(4): 1146-52.
39. Molderings et al. Journal of Hematology & Oncology 2011, 4:10
40. Tanegashima et al. (2013), FEBS Lett. 2013 May 11. doi:pii: S0014-5793(13)
41. X1 et al. (2012), Breast Cancer Res Treat. 2012 Oct;135(3):725-35
42. Mishra et al. (2011), J Leukoc Biol. 2011 Jan;89(1):31-9
43. Tessama et al. (2010), Oncogene. 2010 Sep 16;29(37):5159-70
44. Horny et al. (2007), Pathobiology. 2007;74(2):121-32.
45. Alvarez-Twose et al. (2010), J Allergy Clin Immunol. 2010 Jun; 125(6): 1269-1278
46. St John et al. (2013), Elife. 2013 Apr 30;2:e00481
47. Huang et al. (2013), PLoS One. 2013;8(3):e60827
48. Kosanovic et al. (2013), Histol Histopathol. 2013 Jun;28(6):691-9.
49. Beunk et al. (2013), Expert Rev Clin Immunol. 2013 Jan;9(1):53-63
50. De Vries et al. (2013), Cardiovasc Res. 2013 Feb 1;97(2):311-20
51. Frieri et al. (2013), Curr Allergy Asthma Rep. 2013 Feb;13(1):27-32.
52. Farha et al. (2012), Pulm Circ. 2012 Apr-Jun;2(2):220-8
53. Brockow et al. (2012), Curr Opin Allergy Clin Immunol. 2012 Aug;12(4):354-60.
54. Brown et al. (2012), Front Immunol. 2012;3:147.
55. Summers et al. (2012), Kidney Int. 2012 Sep;82(6):676-85.
56. Park et al. (2011), Int Neurourol J. 2011 Jun;15(2):61-3.
57. Mortaz et al. (2011), Pulm Pharmacol Ther. 2011 Aug;24(4):367-72.
58. Hoffmann et al. (2011), Eur Respir J. 2011 Jun;37(6):1400-10.
59. Kuehn et al. (2010), Curr Protoc Immunol. 2010 Nov;Chapter 7:Unit7.38.
60. Balzar et al. (2011),Am J Respir Crit Care Med. 2011 Feb 1;183(3):299-309.
61. Kalesnikoff et al. (2010),Chem Immunol Allergy. 2010;95:45-66.
62. Matsumori et al. (2010), Circ Res. 2010 May 14;106(9):1533-40.
63. Sun et al. (2009), Circulation. 2009 Sep 15;120(11):973-82.
64. Bargagli et al. (2009), Inflammation. 2009 Oct;32(5):310-4.
65. De Lisle et al. (2009), PLoS One. 2009;4(1):e4283.
66. Holdsworth et al. (2008), J Am Soc Nephrol. 2008 Dec;19(12):2254-61.
67. Navi et al. (2007), Clin Rev Allergy Immunol. 2007 Oct;33(1-2):144-55.#
68. Nistri et al. (2008), Pharmacol Res. 2008 Jan;57(1):43-8. Epub 2007 Nov 9.
69. Baldoma et al. (2009), Int J Cardiol. 2009 Jan 24;131(3):403-4. Epub 2007 Nov 7.
70. Shen (2008), Med Hypotheses. 2008;70(4):760-4. Epub 2007 Oct 4.
71. Theoharides et al. (2007), Adv Exp Med Biol. 2007;601:423-30.
72. Nigrovic 2007), (Immunol Rev. 2007 Jun;217:19-37.
73. Kong et al. (2013), Cell Mol Life Sci. 2013 May 7.
74. Liu et al. (2013), Antioxid Redox Signal. 2013 Apr 15
75. Huang et al. (2013), PLoS One. 2013;8(3):e60827
76. Kennedy et al. (2013), Pharmacol Ther. 2013 Apr;138(1):53-65.
77. Ribatti et al. (2013), Immunol Lett. 2013 May 15. doi:pii: S0165-2478(13)00063-1.
78. Liang et al. (2012), Br J Ophthalmol. 2012 Sep;96(9):1246-51.
79. Bischoff(2007), Nat Rev Immunol. 2007 Feb;7(2):93-104.
80. Bot et al. (2007), Circulation. 2007;115:2516-2525.
81. Robas et al.(2003), J Biol Chem. 2003 Nov 7;278(45):44400-4
82. EP 1 340 979 A2
83. Shaykhiev et al. (2013), Am J Respir Cell Mol Biol. 2013
84. Lv et al. (2013), Mol Med Rep. 2013 Apr;7(4):1245-50
85. Yamamoto et al (2011), Neurosci Lett. 2011 Jan 10;487(3):335-40.

## Claims

1. A method of screening for an antagonist of a Mrg receptor comprising the steps of
a. contacting a test compound with a Mrg receptor polypeptide in the presence of a CXCL14 polypeptide,
b. detecting binding of a CXCL14 polypeptide to said Mrg receptor polypeptide in the presence of said test compound,
c. contacting CXCL14 with a Mrg receptor polypeptide in the absence of said test compound, and
d. detecting binding of a CXCL14 polypeptide to said Mrg receptor polypeptide in the absence of said test compound.

2. A method of screening for an antagonist of a Mrg receptor comprising the steps of
a. determining the activity of a Mrg receptor polypeptide in the presence of a CXCL14 polypeptide, and
b. determining the activity of a Mrg receptor polypeptide in the presence of a CXCL14 polypeptide and a test compound.

3. The method of any one of the preceding claims, wherein additionally the binding or activity of a MrgX2 agonist is determined in the presence or absence of said test compound is determined.

4. The method according to claim 3, wherein the MrgX2 agonist is comprised in the group of agonists consisting of LL37, PAMP, BAM22, Cortistatin 17, Somatostatin (different isoforms), NPFF, Oxytocin, Cyclosomatostatin, Dynorphin A, [Arg8] vasopressin, Substance P and Hexarelin.

5. A method of screening for an agonist of a Mrg receptor comprising the steps of
a. generating a CXCL14 variant by replacing one or more amino acids of a CXCL14 polypeptide by another naturally or non-naturally occurring amino acid, and
b. determining the activity of a Mrg receptor polypeptide in the presence of a CXCL14 variant.

6. The method of any one of the preceding claims, wherein the Mrg receptor is MrgX2.

7. The method of any one of the preceding claims, wherein the Mrg receptor is human MrgX2.

8. The method of any one of the preceding claims, wherein CXCL14 polypeptide is human CXCL14 polypeptide.

9. The method of any one of the preceding claims, wherein CXCL14 polypeptide is depicted in SEQ ID NO: 1.

## Patentansprüche

1. Screening-Verfahren für einen Antagonisten eines Mrg-Rezeptors, umfassend die Schritte
a. Inkontaktbringen einer Testverbindung mit einem Mrg-Rezeptor-Polypeptid in Gegenwart eines CXCL14-Polypeptids,
b. Nachweisen des Bindens eines CXCL14-Polypeptids an das Mrg-Rezeptor-Polypeptid in Gegenwart der Testverbindung,
c. Inkontaktbringen von CXCL14 mit einem Mrg-Rezeptor-Polypeptid in Abwesenheit der Testverbindung und
d. Nachweisen des Bindens eines CXCL14-Polypeptids an das Mrg-Rezeptor-Polypeptid in Abwesenheit der Testverbindung.

2. Screening-Verfahren für einen Antagonisten eines Mrg-Rezeptors, umfassend die Schritte
a. Bestimmen der Aktivität eines Mrg-Rezeptor-Polypeptids in Gegenwart eines CXCL14-Polypeptids und
b. Bestimmen der Aktivität eines Mrg-Rezeptor-Polypeptids in Gegenwart eines CXCL14-Polypeptids und einer Testverbindung.

3. Verfahren nach einem der vorhergehenden Ansprüche, wobei zusätzlich die Bindung oder Aktivität eines MrgX2-Agonisten in der Gegenwart bestimmt wird oder Abwesenheit der Testverbindung bestimmt wird.

4. Verfahren gemäß Anspruch 3, wobei der MrgX2-Agonist in der aus LL37, PAMP, BAM22, Cortistatin 17, Somatostatin (unterschiedliche Isoformen), NPFF, Oxytocin, Cyclosomatostatin, Dynorphin A, [Arg8]-Vasopressin, Substance P und Hexarelin bestehenden Gruppe von Agonisten umfasst ist.

5. Screening-Verfahren für einen Agonisten eines Mrg-Rezeptors, umfassend die Schritte
a. Erzeugen einer CXCL14-Variante, indem eine oder mehrere Aminosäuren eines CXCL14-Polypeptids durch eine andere natürlich oder nicht natürlich vorkommende Aminosäure ersetzt werden, und
b. Bestimmen der Aktivität eines Mrg-Rezeptor-Polypeptids in Gegenwart einer CXCL14-Variante.

6. Verfahren nach einem der vorhergehenden Ansprüche, wobei es sich bei dem Mrg-Rezeptor um MrgX2 handelt.

7. Verfahren nach einem der vorhergehenden Ansprüche, wobei es sich bei dem Mrg-Rezeptor um menschlichen MrgX2 handelt.

8. Verfahren nach einem der vorhergehenden Ansprüche, wobei es sich bei CXCL14-Polypeptid um menschliches CXCL14-Polypeptid handelt.

9. Verfahren nach einem der vorhergehenden Ansprüche, wobei CXCL14-Polypeptid unter SEQ ID NO: 1 dargestellt ist.

## Revendications

1. Procédé de criblage d'un antagoniste d'un récepteur de Mrg comprenant les étapes de
a. mise en contact d'un composé d'essai avec un polypeptide de récepteur de Mrg en présence d'un polypeptide CXCL14,
b. détection de la liaison d'un polypeptide CXCL14 audit polypeptide de récepteur de Mrg en présence dudit composé d'essai,
c. mise en contact de CXCL14 avec un polypeptide de récepteur de Mrg en l'absence dudit composé d'essai, et
d. détection de la liaison d'un polypeptide CXCL14 audit polypeptide de récepteur de Mrg en l'absence dudit composé d'essai.

2. Procédé de criblage d'un antagoniste d'un récepteur de Mrg comprenant les étapes de
a. détermination de l'activité d'un polypeptide de récepteur de Mrg en présence d'un polypeptide CXCL14, et
b. détermination de l'activité d'un polypeptide de récepteur de Mrg en présence d'un polypeptide CXCL14 et d'un composé d'essai.

3. Procédé de l'une quelconque des revendications précédentes, dans lequel, en outre, la liaison ou l'activité d'un agoniste de MrgX2 est déterminée en présence ou en l'absence dudit composé d'essai est déterminée.

4. Procédé selon la revendication 3, dans lequel l'agoniste de MrgX2 est compris dans le groupe d'agonistes constitué des LL37, PAMP, BAM22, cortistatine 17, somatostatine (différentes isoformes), NPFF, oxytocine, cyclosomatostatine, dynorphine A, [Arg8] vasopressine, substance P et hexaréline.

5. Procédé de criblage d'un agoniste d'un récepteur de Mrg comprenant les étapes de
a. génération d'un variant de CXCL14 par remplacement d'un ou plusieurs acides aminés d'un polypeptide CXCL14 par un autre acide aminé d'origine naturelle ou non naturelle, et
b. détermination de l'activité d'un polypeptide de récepteur de Mrg en présence d'un variant de CXCL14.

6. Procédé de l'une quelconque des revendications précédentes, dans lequel le récepteur de Mrg est MrgX2.

7. Procédé de l'une quelconque des revendications précédentes, dans lequel le récepteur de Mrg est MrgX2 humain.

8. Procédé de l'une quelconque des revendications précédentes, dans lequel le polypeptide CXCL14 est un polypeptide CXCL14 humain.

9. Procédé de l'une quelconque des revendications précédentes, dans lequel le polypeptide CXCL14 est décrit dans SEQ ID NO: 1.


### SEQUENCE LISTING

<110> Bayer Pharma AG
<120> Mrg receptor modulators
<130> BHC 13 1 026
<160> 11
<170> PatentIn version 3.5
<210> 1
   <211> 77
   <212> PRT
   <213> Homo sapiens
<400> 1
   Ser Lys Cys Lys Cys Ser Arg Lys Gly Pro Lys Ile Arg Tyr Ser Asp
   1               5                   10                  15
   Val Lys Lys Leu Glu Met Lys Pro Lys Tyr Pro His Cys Glu Glu Lys
               20                  25                  30
   Met Val Ile Ile Thr Thr Lys Ser Val Ser Arg Tyr Arg Gly Gln Glu
           35                  40                  45
   His Cys Leu His Pro Lys Leu Gln Ser Thr Lys Arg Phe Ile Lys Trp
       50                  55                  60
   Tyr Asn Ala Trp Asn Glu Lys Arg Arg Val Tyr Glu Glu
   65                  70                  75
<210> 2
   <211> 1989
   <212> DNA
   <213> Homo sapiens
<400> 2
   aatgtgtgcg cgctgtggta tgggtgtgca agtgtgcgaa ggcggcgtgt tgtgtgagcg      60
   agagggtagc ggatgtgtgt gtgcgtgtgc gcgcgtggct ccgggtgtgc gccgctgcga     120
   tagcgggtcc tttcccgggg cgggcgacgg gcgggctggg aaggtctcct cccctcacca     180
   cattgagaaa tctcagtgag tcaccgagtg gttctgcata ttaatgagct cgctcgctgc     240
   gagggcagga gcggatttaa aagaggccag ggcgggcgga gggaggctgt ggagagagcg     300
   cggagacaag cgcagagcgc agcgcacggc cacagacagc cctgggcatc caccgacggc     360
   gcagccggag ccagcagagc cggaaggcgc gccccgggca gagaaagccg agcagagctg     420
   ggtggcgtct ccgggccgcc gctccgacgg gccagcgccc tccccatgtc cctgctccca     480
   cgccgcgccc ctccggtcag catgaggctc ctggcggccg cgctgctcct gctgctgctg     540
   gcgctgtaca ccgcgcgtgt ggacgggtcc aaatgcaagt gctcccggaa gggacccaag 600
   atccgctaca gcgacgtgaa gaagctggaa atgaagccaa agtacccgca ctgcgaggag 660
   aagatggtta tcatcaccac caagagcgtg tccaggtacc gaggtcagga gcactgcctg 720
   caccccaagc tgcagagcac caagcgcttc atcaagtggt acaacgcctg gaacgagaag 780
   cgcagggtct acgaagaata gggtgaaaaa cctcagaagg gaaaactcca aaccagttgg 840
   gagacttgtg caaaggactt tgcagattaa aaaaaaaaaa aaaaaaaaaa aaaaaaaaaa 900
   aaaaaaaaaa agcctttctt tctcacaggc ataagacaca aattatatat tgttatgaag 960
   cactttttac caacggtcag tttttacatt ttatagctgc gtgcgaaagg cttccagatg 1020
   ggagacccat ctctcttgtg ctccagactt catcacaggc tgctttttat caaaaagggg 1080
   aaaactcatg cctttccttt ttaaaaaatg cttttttgta tttgtccata cgtcactata 1140
   catctgagct ttataagcgc ccgggaggaa caatgagctt ggtggacaca tttcattgca 1200
   gtgttgctcc attcctagct tgggaagctt ccgcttagag gtcctggcgc ctcggcacag 1260
   ctgccacggg ctctcctggg cttatggccg gtcacagcct cagtgtgact ccacagtggc 1320
   ccctgtagcc gggcaagcag gagcaggtct ctctgcatct gttctctgag gaactcaagt 1380
   ttggttgcca gaaaaatgtg cttcattccc ccctggttaa tttttacaca ccctaggaaa 1440
   catttccaag atcctgtgat ggcgagacaa atgatcctta aagaaggtgt ggggtctttc 1500
   ccaacctgag gatttctgaa aggttcacag gttcaatatt taatgcttca gaagcatgtg 1560
   aggttcccaa cactgtcagc aaaaacctta ggagaaaact taaaaatata tgaatacatg 1620
   cgcaatacac agctacagac acacattctg ttgacaaggg aaaaccttca aagcatgttt 1680
   ctttccctca ccacaacaga acatgcagta ctaaagcaat atatttgtga ttccccatgt 1740
   aattcttcaa tgttaaacag tgcagtcctc tttcgaaagc taagatgacc atgcgccctt 1800
   tcctctgtac atataccctt aagaacgccc cctccacaca ctgcccccca gtatatgccg 1860
   cattgtactg ctgtgttata tgctatgtac atgtcagaaa ccattagcat tgcatgcagg 1920
   tttcatattc tttctaagat ggaaagtaat aaaatatatt tgaaatgtac caaaaaaaaa 1980
   aaaaaaaaa 1989
<210> 3
   <211> 330
   <212> PRT
   <213> Homo sapiens
<400> 3
   Met Asp Pro Thr Thr Pro Ala Trp Gly Thr Glu Ser Thr Thr Val Asn
   1               5                   10                  15
   Gly Asn Asp Gln Ala Leu Leu Leu Leu Cys Gly Lys Glu Thr Leu Ile
               20                  25                  30
   Pro Val Phe Leu Ile Leu Phe Ile Ala Leu Val Gly Leu Val Gly Asn
           35                  40                  45
   Gly Phe Val Leu Trp Leu Leu Gly Phe Arg Met Arg Arg Asn Ala Phe
       50                  55                  60
   Ser Val Tyr Val Leu Ser Leu Ala Gly Ala Asp Phe Leu Phe Leu Cys
   65                  70                  75                  80
   Phe Gln Ile Ile Asn Cys Leu Val Tyr Leu Ser Asn Phe Phe Cys Ser
                   85                  90                  95
   Ile Ser Ile Asn Phe Pro Ser Phe Phe Thr Thr Val Met Thr Cys Ala
               100                 105                 110
   Tyr Leu Ala Gly Leu Ser Met Leu Ser Thr Val Ser Thr Glu Arg Cys
           115                 120                 125
   Leu Ser Val Leu Trp Pro Ile Trp Tyr Arg Cys Arg Arg Pro Arg His
       130                 135                 140
   Leu Ser Ala Val Val Cys Val Leu Leu Trp Ala Leu Ser Leu Leu Leu
   145                 150                 155                 160
   Ser Ile Leu Glu Gly Lys Phe Cys Gly Phe Leu Phe Ser Asp Gly Asp
                   165                 170                 175
   Ser Gly Trp Cys Gln Thr Phe Asp Phe Ile Thr Ala Ala Trp Leu Ile
               180                 185                 190
   Phe Leu Phe Met Val Leu Cys Gly Ser Ser Leu Ala Leu Leu Val Arg
           195                 200                 205
   Ile Leu Cys Gly Ser Arg Gly Leu Pro Leu Thr Arg Leu Tyr Leu Thr
       210                 215                 220
   Ile Leu Leu Thr Val Leu Val Phe Leu Leu Cys Gly Leu Pro Phe Gly
   225                 230                 235                 240
   Ile Gln Trp Phe Leu Ile Leu Trp Ile Trp Lys Asp Ser Asp Val Leu
                   245                 250                 255
   Phe Cys His Ile His Pro Val Ser Val Val Leu Ser Ser Leu Asn Ser
               260                 265                 270
   Ser Ala Asn Pro Ile Ile Tyr Phe Phe Val Gly Ser Phe Arg Lys Gln
           275                 280                 285
   Trp Arg Leu Gln Gln Pro Ile Leu Lys Leu Ala Leu Gln Arg Ala Leu
       290                 295                 300
   Gln Asp Ile Ala Glu Val Asp His Ser Glu Gly Cys Phe Arg Gln Gly
   305                 310                 315                 320
   Thr Pro Glu Met Ser Arg Ser Ser Leu Val
                   325                 330
<210> 4
   <211> 2131
   <212> DNA
   <213> Homo sapiens
<400> 4
   gtttgccagt cccaggaaag cacttctcaa ctcaccaact ccagtagaaa gaagggtgtt      60
   aaggggcacc agtggaggtt ttctgagcat ggatccaacc accccggcct ggggaacaga     120
   aagtacaaca gtgaatggaa atgaccaagc ccttcttctg ctttgtggca aggagaccct     180
   gatcccggtc ttcctgatcc ttttcattgc cctggtcggg ctggtaggaa acgggtttgt     240
   gctctggctc ctgggcttcc gcatgcgcag gaacgccttc tctgtctacg tcctcagcct     300
   ggccggggcc gacttcctct tcctctgctt ccagattata aattgcctgg tgtacctcag     360
   taacttcttc tgttccatct ccatcaattt ccctagcttc ttcaccactg tgatgacctg     420
   tgcctacctt gcaggcctga gcatgctgag caccgtcagc accgagcgct gcctgtccgt     480
   cctgtggccc atctggtatc gctgccgccg ccccagacac ctgtcagcgg tcgtgtgtgt     540
   cctgctctgg gccctgtccc tactgctgag catcttggaa gggaagttct gtggcttctt     600
   atttagtgat ggtgactctg gttggtgtca gacatttgat ttcatcactg cagcgtggct     660
   gattttttta ttcatggttc tctgtgggtc cagtctggcc ctgctggtca ggatcctctg     720
   tggctccagg ggtctgccac tgaccaggct gtacctgacc atcctgctca cagtgctggt     780
   gttcctcctc tgcggcctgc cctttggcat tcagtggttc ctaatattat ggatctggaa     840
   ggattctgat gtcttatttt gtcatattca tccagtttca gttgtcctgt catctcttaa     900
   cagcagtgcc aaccccatca tttacttctt cgtgggctct tttaggaagc agtggcggct     960
   gcagcagccg atcctcaagc tggctctcca gagggctctg caggacattg ctgaggtgga    1020
   tcacagtgaa ggatgcttcc gtcagggcac cccggagatg tcgagaagca gtctggtgta    1080
   gagatggaca gcctctactt ccatcagata tatgtggctt tgagaggcaa ctttgcccct    1140
   gtctgtctga tttgctgaac tttctcagtc ctgattttaa aacagttaag agagtccttg    1200
   tgaggattaa gtgagacagt gcctatgaaa caaacactaa gtgcagtgtc tctggaactg    1260
   ccttactcac aggcttccac cacagcccta tgagagcttt gccaactctg cggtccatga 1320
   ctgttcccac attttaatga atcctacctt tcgcagaagg ctgaaagcag ggcagaaaag 1380
   atctacattt ctttggacac tgcacttgat agggactcaa agaatgttat atttttaatt 1440
   aatttctttt tctcttccgt acaatttctg tctcaacaaa attagaagaa ttaaatttaa 1500
   aactagctcc aaaagagcag tcgtctttca ttttggcaga ccttagaata tccccctagc 1560
   ttaataaatc tttgttgaat ggcttaatga atgaataaac tggttaatgt ttaagttaaa 1620
   cctctgaaaa gtctccattt accagatttg agtcactaaa tttattgctt tcactacttt 1680
   tgaattttgc aaacatgaaa ttaagtttta taattagata aatcaatgtc aacacatatt 1740
   taaagtttga ggtacactgt cttcctgtgg tttcctttca catgccatcc cttaaaatcc 1800
   cagctacacg ccttcccatt ccttcccctt tgcctttgtt ctaatcttcc ctctctgggg 1860
   gctctctaat tcgtcctgga agtttccagt ggtcttatag actccatgtt cttggaggac 1920
   aggctgtatg tcagatttac cttttattcc gaagaactcg gagcatttat tttgttaatt 1980
   aaattgcaca tatttttaaa agttacgtgt tccacagaat aaaatattaa ttgtaaaaaa 2040
   aaaaaaaaaa aaaaaaaaaa aaaaaaaaaa aaaaaaaaaa aaaaaaaaaa aaaaaaaaaa 2100
   aaaaaaaaaa aaaaaaaaaa aaaaaaaaaa a 2131
<210> 5
   <211> 21
   <212> DNA
   <213> Artificial Sequence
<220>
   <223> primer
<400> 5
   caggaacgcc ttctctgtct a 21
<210> 6
   <211> 18
   <212> DNA
   <213> Artificial Sequence
<220>
   <223> primer
<400> 6
   cgtcctcagc ctggccgg 18
<210> 7
   <211> 20
   <212> DNA
   <213> Artificial Sequence
<220>
   <223> primer
<400> 7
   tggaagcaga ggaagaggaa 20
<210> 8
   <211> 20
   <212> DNA
   <213> Artificial Sequence
<220>
   <223> primer
<400> 8
   aagttcatcc ggcaccagtc 20
<210> 9
   <211> 22
   <212> DNA
   <213> Artificial Sequence
<220>
   <223> primer
<400> 9
   cccagaggca ttgacaacag gg 22
<210> 10
   <211> 21
   <212> DNA
   <213> Artificial Sequence
<220>
   <223> primer
<400> 10
   tggcccttga atcttctacg a 21
<210> 11
   <211> 99
   <212> PRT
   <213> Mus musculus
<400> 11
   Met Arg Leu Leu Ala Ala Ala Leu Leu Leu Leu Leu Leu Ala Leu Cys
   1               5                   10                  15
   Val Arg Ala Leu Asp Gly Ser Lys Cys Lys Cys Ser Arg Lys Gly Pro
               20                  25                  30
   Lys Ile Arg Tyr Ser Asp Val Lys Lys Leu Glu Met Lys Pro Lys Tyr
           35                  40                  45
   Pro His Cys Glu Glu Lys Met Val Ile Val Thr Thr Lys Ser Met Ser
       50                  55                  60
   Arg Tyr Arg Gly Gln Glu His Cys Leu His Pro Lys Leu Gln Ser Thr
   65                  70                  75                  80
   Lys Arg Phe Ile Lys Trp Tyr Asn Ala Trp Asn Glu Lys Arg Arg Val
                   85                  90                  95
   Tyr Glu Glu